# EUROPÄISCHE PATENTSCHRIFT

(11) **EP 3 372 096 B1**
(45) Veröffentlichungstag und Bekanntmachung des Hinweises auf die Patenterteilung: **28.04.2021**
(21) Anmeldenummer: 18159768.3
(22) Anmeldetag: 02.03.2018
(51) Int. Cl.: F04B 17/03, F04B 23/04, A24F 47/00, A61M 15/06, A61M 11/04, F04B 19/00, F04B 43/04

(54) **VERDAMPFEREINHEIT FÜR EINEN INHALATOR**
EVAPORATOR UNIT FOR AN INHALER
UNITÉ D'ÉVAPORATION POUR UN INHALATEUR

(30) Priorität: 06.03.2017 DE 102017104555
(43) Veröffentlichungstag der Anmeldung: 12.09.2018
(73) Patentinhaber: Hauni Maschinenbau GmbH, 21033 Hamburg (DE)
(72) Erfinder: SCHMIDT, Rene, 21244 Buchholz i.d.N. (DE); KESSLER, Marc, 22415 Hamburg (DE)
(74) Vertreter: Müller Verweyen

(56) Entgegenhaltungen:
- EP-A1- 0 539 674
- EP-A2- 2 397 177
- WO-A1-01/21319
- WO-A1-2012/114230
- US-A1- 2014 352 689

## Beschreibung

Die vorliegende Erfindung betrifft eine Verdampfereinheit für einen Inhalator, mit einem Verdampfer, der ein Heizelement zum Verdampfen von durch den Verdampfer geförderter Flüssigkeit aufweist.

Bei der Verdampfung von E-Liquids in herkömmlichen Docht-Wendel-Verdampfern mit Nachströmen von frischem Liquid aus dem Tank stellt sich im Docht eine mit Glycerin angereicherte Zusammensetzung ein, bei der die Gasphasenzusammensetzung der des nachströmenden Liquids entspricht. Dazu gehört eine entsprechende Siedetemperatur. Im Docht wird sich dabei eine mit Glycerin angereicherte Zusammensetzung am Rand einstellen, die entlang des Dochtes höchstwahrscheinlich nicht konstant ist. Es lässt sich zeigen, dass in diesem Fall in Teilbereichen des Dochtes höhere Siedetemperaturen vorkommen müssen, als wenn das Liquid im Bereich der Dampfbildung eine konstante Zusammensetzung hat, d.h. gut durchmischt ist. Des Weiteren kann ein Zusammensetzungsgradient im Dochtquerschnitt zu einem schlecht zu kontrollierenden spontanen Verdampfen von Liquid in der Dochtmitte führen, was zu einem Mitreißen von Liquidtropfen führt. Im Sinne einer möglichst gleichmäßigen Verdampfung bei möglichst geringer Temperatur, d.h. Vermeidung von thermischer Zersetzung des Liquids, sollte dieser Zustand vermieden werden.

Die WO 01/21319 A1 offenbart eine Verdampfereinheit mit den Merkmalen des Oberbegriffs von Anspruch 1.

Die Aufgabe der Erfindung besteht darin, eine Verdampfereinheit bereitzustellen, bei der insbesondere das Liquid im Bereich der Dampfbildung eine möglichst homogene Zusammensetzung hat.

Die Erfindung löst diese Aufgabe mit den Merkmalen der unabhängigen Ansprüche. Erfindungsgemäß weist die Verdampfereinheit eine Kreislaufleitung auf, in der der Verdampfer angeordnet ist, wobei in der Kreislaufleitung eine Umwälzeinrichtung zum Umwälzen von Flüssigkeit durch die Kreislaufleitung angeordnet ist. Während der erfindungsgemäßen Umwälzung wird eine permanente Vermischung der zu verdampfenden Flüssigkeit erreicht, wodurch die Zusammensetzung der umgewälzten Flüssigkeit im Wesentlichen konstant gehalten wird. Aufgrund der erfindungsgemäßen Anordnung des Verdampfers in der Kreislaufleitung wird erreicht, dass die konstante Flüssigkeitszusammensetzung bis in den Bereich der Dampfbildung hinein erzielt wird, so dass auch das vorrangige Ziel einer konstanten Dampfzusammensetzung erreicht wird. Insbesondere zur Vermeidung großer lokaler Zusammensetzungsgradienten erfolgt demnach erfindungsgemäß eine Zwangskonvektion des in der Kreislaufleitung eingeschlossenen Liquidvolumens.

Vorzugsweise ist die Umwälzeinrichtung elektrisch angetrieben, da ein elektrischer Energiespeicher zum Betrieb des Heizelements in dem Verdampfer in der Regel ohnehin vorhanden ist. Die Umwälzeinrichtung kann insbesondere eine (elektrisch angetriebene) Pumpe und vorzugsweise eine Mikropumpe sein.

Zur Aufrechterhaltung der Funktion der Verdampfereinheit muss verdampfte Flüssigkeit aus dem Flüssigkeitsspeicher zu dem Verdampfer nachgefördert werden. Zum Nachfördern von verdampfter Flüssigkeit ist daher erfindungsgemäß ein Kapillarförderer, eine Förderpumpe, eine Druckbeaufschlagungseinrichtung oder ein anderer geeigneter Fördermechanismus vorgesehen.

Der Flüssigkeitsspeicher ist vorteilhaft in der Kreislaufleitung angeordnet, so dass die in dem Flüssigkeitsspeicher gespeicherte Flüssigkeit mit umgewälzt wird. Dies kann zur Erreichung des Ziels einer möglichst konstanten Flüssigkeitszusammensetzung erheblich beitragen. Jedoch ist es nicht zwingend erforderlich, dass der Flüssigkeitsspeicher in der Kreislaufleitung angeordnet ist. In anderen Ausführungsformen ist der Flüssigkeitsspeicher separat von der Kreislaufleitung angeordnet. Die Verdampfereinheit weist dann vorteilhaft ein Verzweigungselement zum Zuführen von Flüssigkeit aus dem Flüssigkeitsspeicher in die Kreislaufleitung auf. Das Verzweigungselement ist vorzugsweise als Mischer, insbesondere als statischer Mischer, ausgebildet und kann beispielsweise ein Labyrinthmischer sein.

Die Verdampfereinheit ist zur Vermeidung hoher thermischer Kapazitäten und damit verbundenem schlechtem Ansprechverhalten vorteilhaft möglichst klein ausgeführt. Der Verdampfer und/oder die Verdampfereinheit sind daher vorzugsweise auf der Grundlage von MEMS-Technologie gefertigt und somit vorteilhaft ein einheitliches Mikro-Elektro-Mechanisches System.

Die Erfindung wird im Folgenden anhand bevorzugter Ausführungsformen unter Bezugnahme auf die beigefügten Figuren erläutert. Dabei zeigt
- Fig. 1: eine Querschnittsansicht eines elektronischen Zigarettenprodukts in einer Ausführungsform der Erfindung;
- Fig. 2: eine Querschnittsansicht einer Kartusche für ein elektronisches Zigarettenprodukt; und
- Fig. 3-7: eine schematische Darstellung einer Verdampfereinheit in unterschiedlichen Ausführungsformen der Erfindung.

Das elektronische Zigarettenprodukt 10 umfasst ein im Wesentlichen stabförmiges oder zylindrisches Gehäuse 11. In dem Gehäuse 11 ist ein Luftkanal 30 zwischen mindestens einer Lufteinlassöffnung 31 und dem Mundende 32 des Zigarettenprodukts 10 vorgesehen. Das Mundende 32 des Zigarettenprodukts 10 bezeichnet dabei das Ende, an dem der Konsument zwecks Inhalation zieht und dadurch das Zigarettenprodukt 10 mit einem Unterdruck beaufschlagt und eine Luftströmung 34 in dem Luftkanal 30 erzeugt. Mindestens eine Lufteinlassöffnung 31 kann an der Mantelseite des Gehäuses 11 angeordnet sein. Zusätzlich oder alternativ kann mindestens eine Lufteinlassöffnung 31A am entfernten Ende 33 des Zigarettenprodukts 10 angeordnet sein. Das entfernte Ende 33 des Zigarettenprodukts 10 bezeichnet das dem Mundende 32 entgegengesetzte Ende des Zigarettenprodukts 10.

Nach einem oder mehreren Lufteinlässen 31, 31A kann im Strömungsweg der Luftströmung 34 vorteilhaft eine Lufterwärmungseinrichtung 37 zum Erwärmen bzw. Vorwärmen der eintretenden Luft angeordnet sein. Hierdurch kann die Aerosolbildung optimiert werden. Die Lufterwärmungseinrichtung 37 kann beispielsweise benachbart zu der Energieversorgungseinheit 14 angeordnet sein und/oder sich in Umfangsrichtung um die Mantelinnenseite des Gehäuses 11 erstrecken.

Die durch die Einlassöffnung 31 angesaugte Luft wird in dem Luftkanal 30, ggf. über die Schnittstelle bzw. Trennfläche 57, zu einem Verdampfer 20 geleitet. Der Verdampfer 20 gibt Flüssigkeit 50 aus dem Flüssigkeitsspeicher 18 als Zugabe 40 in Form kleiner Flüssigkeitstropfen als Nebel/Aerosol und/oder gasförmig als Dampf in den Luftstrom 34 zu. Ein vorteilhaftes Volumen des Flüssigkeitsspeichers 18 liegt im Bereich zwischen 0,1 ml und 5 ml, vorzugsweise zwischen 0,5 ml und 3 ml, weiter vorzugsweise zwischen 0,7 ml und 2 ml oder 1,5 ml.

Das Zigarettenprodukt 10 umfasst, vorteilhaft am entfernten Ende 33 des Zigarettenprodukts 10, eine elektronische Energieversorgungseinheit 12 mit einem elektrischen Energiespeicher 14 und einer elektrischen/elektronischen Steuerungseinrichtung 15. Der Energiespeicher 14 kann insbesondere eine elektrochemische Einweg-Batterie oder ein wiederaufladbarer elektrochemischer Akku, z.B. ein Li-Ionen-Akku, sein. Das Zigarettenprodukt 10 umfasst des Weiteren, vorteilhaft am Mundende 32 des Zigarettenprodukts 10, eine Verdampfereinheit 17 mit einem Flüssigkeitsspeicher 18, einer elektrischen Steuerungseinrichtung 19 und dem Verdampfer 20.

Anstelle der getrennten elektrischen/elektronischen Steuerungseinrichtung 15, 19 kann auch eine einheitliche elektrische/elektronische Steuerungseinrichtung vorgesehen sein, die entweder in der Energieversorgungseinheit 12 oder in der Verdampfereinheit 17 angeordnet sein kann. Die Gesamtheit der elektrischen/elektronischen Steuerungseinrichtungen des Zigarettenprodukts 10 wird im Folgenden als Steueranordnung 29 bezeichnet.

In dem Gehäuse 11 ist vorteilhaft ein Sensor, beispielsweise ein Drucksensor oder ein Druck- oder Strömungsschalter, angeordnet, wobei die Steueranordnung auf der Grundlage eines von dem Sensor ausgegebenen Sensorsignals einen Betriebszustand des Zigarettenprodukts 10, in dem ein Konsument am Mundende 32 des Zigarettenprodukts 10 zieht, um zu inhalieren, feststellen kann. In diesem Betriebszustand steuert die Steueranordnung 29 den Verdampfer 20 an, um Flüssigkeit aus dem Flüssigkeitsspeicher 18 als Zugabe 40 in Form kleiner Flüssigkeitstropfen als Nebel/Aerosol und/oder gasförmig als Dampf in den Luftstrom 34 zuzugeben.

Die in dem Flüssigkeitsspeicher 18 gespeicherte, zu dosierende Flüssigkeit (d.h. das flüssige Komponentengemisch) ist beispielsweise eine Mischung aus 1,2-Propylenglykol, Glycerin und/oder Wasser, der ein oder mehrere Aromen (Flavour) und/oder Wirkstoffe, wie beispielsweise Nikotin, zugemischt sein können.

Die Verdampfereinheit 17 ist vorteilhaft als vom Konsumenten auswechselbare Kartusche 21, d.h. als Einwegteil ausgeführt. Der insbesondere den Energiespeicher 14 enthaltende Rest des Zigarettenprodukts 10 ist vorteilhaft als vom Konsumenten wiederverwendbares Grundteil 56, d.h. als Mehrwegteil ausgeführt. Die Kartusche 21 ist vom Konsumenten mit dem Grundteil 56 verbindbar und vom Grundteil 56 lösbar ausgebildet. Zwischen der Kartusche 21 und dem wiederverwendbaren Grundteil 56 ist somit eine Trennfläche bzw. Schnittstelle 57 gebildet. Das Kartuschengehäuse 58 kann einen Teil des Gehäuses 11 des Zigarettenprodukts 10 bilden.

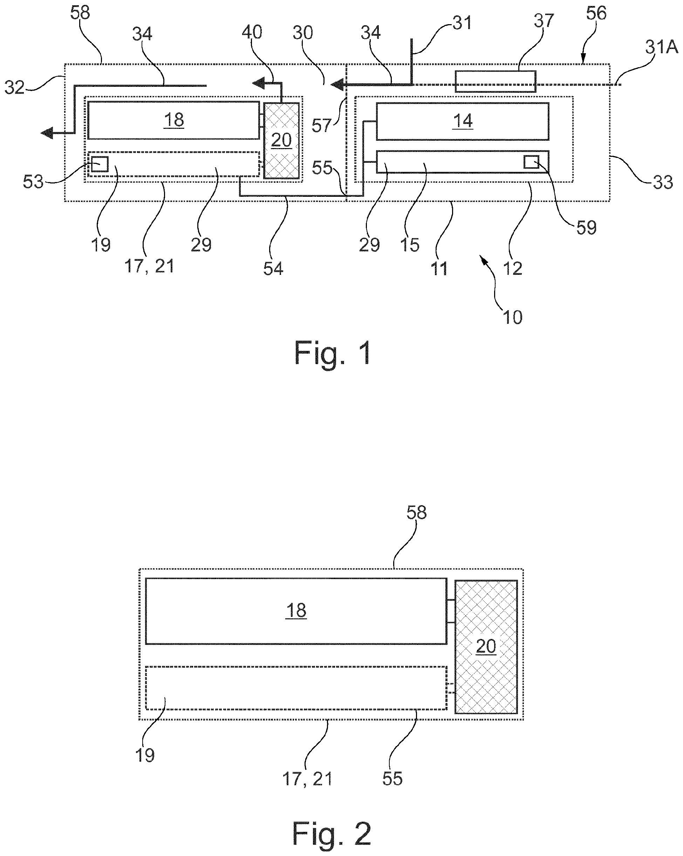

In anderen Ausführungsformen, siehe Fig. 2, ist die Verdampfereinheit 17 als Kartusche 21 ausgeführt, die in den wiederverwendbaren Grundteil 56 des Zigarettenprodukts 10 durch den Konsumenten einsetzbar und aus diesem entnehmbar ist. Das Kartuschengehäuse 58 ist in diesem Fall ein von dem Gehäuse 11 des Zigarettenprodukts 10 separates Gehäuse.

Die Kartusche 21 umfasst mindestens den Flüssigkeitsspeicher 18. Die Kartusche 21 kann, wie in Fig. 2 gezeigt, die elektrische/elektronische Steuereinrichtung 19 umfassen. In anderen Ausführungsformen ist die elektrische/elektronische Steuereinrichtung 19 ganz oder teilweise fester Bestandteil des Grundteils 56. Ebenso kann der Verdampfer 20 Teil der Kartusche 21 oder in dem Grundteil 56 angeordnet sein. Die Kartusche 21 kann daher in manchen Ausführungsformen im Wesentlichen nur aus dem Flüssigkeitsspeicher 18 bestehen und ggf. dem Kartuschengehäuse 58, wobei das Kartuschengehäuse 58 alternativ von dem Gehäuse des Flüssigkeitsspeichers 18 gebildet sein kann, so dass ein separates Kartuschengehäuse 58 entbehrlich sein kann.

Die Verdampfereinheit 17 kann neben der Verwendung in stabförmigen Zigarettenprodukten 10 auch in anderen Inhalatoren eingesetzt werden, beispielsweise in einer elektronischen Pfeife, Shisha, in Heat-not-burn-Produkten, oder einem medizinischen Inhalator. Der Energiespeicher 14 ist in der Regel nicht Teil der Kartusche 21, sondern Teil des wiederverwendbaren Grundteils 56.

Die Verdampfereinheit 17 bzw. die Kartusche 21 umfasst vorteilhaft einen nichtflüchtigen Informationsspeicher 53 (siehe Fig. 1) zum Speichern von die Verdampfereinheit 17 bzw. die Kartusche 21 betreffender Information bzw. Parametern, beispielsweise in Ausführung als EEPROM, RFID oder anderer geeigneter Form. Der Informationsspeicher 53 kann Teil der elektrischen/elektronischen Steuereinrichtung 19 oder separat davon ausgebildet sein. In dem Informationsspeicher 53 gespeichert ist vorteilhaft Information zum Inhaltsstoff, d.h. zur Zusammensetzung der in dem Flüssigkeitsspeicher 18 gespeicherten Flüssigkeit; Information zum Prozessprofil, insbesondere Leistungs-/Temperatursteuerung; Daten zur Zustandsüberwachung bzw. Systemprüfung, beispielsweise Dichtigkeitsprüfung; Daten betreffend Kopierschutz und Fälschungssicherheit, insbesondere umfassend eine ID zur eindeutigen Kennzeichnung der Verdampfereinheit 17 bzw. Kartusche 21; Seriennummer, Herstelldatum und/ oder Ablaufdatum; und/oder Zugzahl (Anzahl der Inhalationszüge durch den Konsumenten) bzw. der Nutzungszeit. Der Datenspeicher 53 ist vorteilhaft über Kontakte und/oder Leitungen mit der Steuereinrichtung 15 des Grundteils 56 verbunden oder verbindbar.

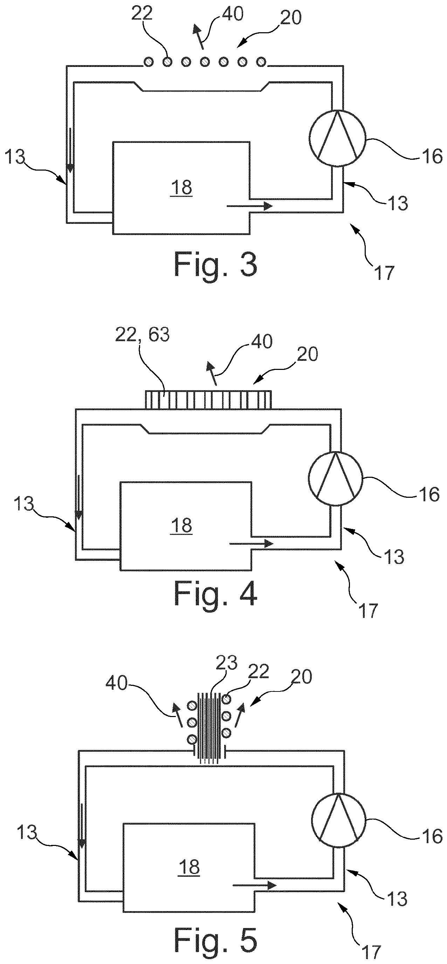

Vorteilhafte Ausführungsformen erfindungsgemäßer Verdampfereinheiten 17 sind in den Figuren 3 bis 7 gezeigt.

Der Verdampfer 20 umfasst ein elektrisches Heizelement 22, insbesondere ein Widerstandsheizelement, zum Erhitzen und somit Verdampfen von das Heizelement 22 kontaktierender Flüssigkeit. Das Heizelement wird von in dem Energiespeicher 14 erzeugtem Strom durchflossen und erhitzt sich dadurch. Der Heizstrom und somit der zeitliche Verlauf des Heizvorgang wird vorzugsweise durch die elektronische Steuerungsanordnung 29 gesteuert. In der Ausführungsform gemäß den Figuren 3, 6 und 7 kann das Heizelement 22 beispielsweise ein Spiralheizelement aus einem Widerstandsdraht sein. Die Ausführung der Heizelemente 22 in den Figuren 4 und 5 wird weiter unten beschrieben.

Die Verdampfereinheit 17 umfasst die erfindungsgemäße Ringleitung bzw. Kreislaufleitung 13, in der der Verdampfer 20 und eine elektrisch angetriebene Umwälzeinrichtung 16 angeordnet sind. Die von der Steuereinrichtung 19 bzw. der Steueranordnung 29 gesteuerte Umwälzeinrichtung 16 ist insbesondere eine elektrische (Umwälz-)Pumpe, die vorteilhaft als Mikropumpe ausgeführt ist. Wie aus den Figuren 3 bis 7 ersichtlich, ist die Kreislaufleitung 13 in sich geschlossen, so dass bei laufender Umwälzeinrichtung 16 die Flüssigkeit in der Ringleitung 13 fortlaufend umgewälzt wird, wodurch eine optimale Vermischung der Flüssigkeit und somit ein jederzeit konstantes Mischungsverhältnis erreicht wird.

Demnach erfolgt eine Zwangskonvenktion des in der Kreislaufleitung 13 befindlichen Liquidvolumens, wobei das Liquid am Verdampfer 20 und dem Heizelement 22 vorbeigeführt wird und ein Teil der Flüssigkeit durch Temperatureinwirkung unter Bildung von Dampf/ Aerosol 40 verdampft. Diese Zwangskonvektion wird durch die Umwälzeinrichtung 16 erzeugt und weist einen so großen Massenstrom auf, dass die Konzentrationsänderung durch Verdampfen in dem am Heizelement 22 vorbeigeführten Liquid gering bleibt.

In den bevorzugten Ausführungsformen gemäß Figuren 3 bis 5 ist der Flüssigkeitsspeicher 18 in der Kreislaufleitung 13 angeordnet bzw. in diese eingeschaltet, so dass das gesamte in der Verdampfereinheit befindliche Liquid, einschließlich des Inhalts des Reservoirs 18, durch die Pumpe 16 umgewälzt und an dem Heizelement 22 vorbeigeführt wird.

In den Ausführungsformen gemäß Figuren 3 bis 5 und 7 wird die Nachförderung des verdampften Massenstroms 40 auf geeignete Weise sichergestellt. Dies kann beispielsweise durch kapillare Förderwirkung geschehen. Bei der Ausführungsform gemäß Figur 4 umfasst hierzu der Verdampfer 20 ein blockförmiges Substrat 63 aus einem elektrisch leitenden Material, das mit einer Mehrzahl von Mikrokanälen versehen, die eine Einlassseite des Substrats 63 mit einer Auslassseite flüssigkeitsleitend verbinden. Die Einlassseite ist flüssigkeitsleitend mit der Kreislaufleitung 13 und somit mit dem Flüssigkeitsspeicher 18 verbunden. Aufgrund der Abmessungen der Mikrokanäle wird vorteilhaft eine Kapillarwirkung erzeugt, so dass an der Einlassseite in einen Mikrokanal eindringende Flüssigkeit durch den Mikrokanal nach oben steigt, bis der Mikrokanal mit Flüssigkeit gefüllt ist. Auf diese Weise wird ein selbsttätiger kapillarer Fördermechanismus zum Ausgleich der verdampften Flüssigkeit realisiert.

An das Substrat 63 wird über Elektroden von der Steuereinrichtung 19 eine elektrische Spannung Uh angelegt, die zu einem Stromfluss durch das Substrat 63 führt. Aufgrund des Ohmschen Widerstands des Substrats 63 führt der Stromfluss zu einer Erhitzung des Substrats 63 und daher zu einer Verdampfung von in den Mikrokanälen enthaltener Flüssigkeit, wobei durch spontane Erhitzung die in den Mikrokanälen 62 befindliche Flüssigkeit in Form von Dampf und/oder Aerosol 40 aus den Mikrokanälen 62 getrieben wird. Der auf diese Weise erzeugte Dampf und/ oder Aerosol entweicht zur Auslassseite aus den Mikrokanälen und wird als Dampfzugabe 40 der Luftströmung 34 beigemischt, siehe Figur 1, indem diese außen an den Austrittsöffnungen des Substrats 63 vorbeiströmt.

Bei der Ausführungsform gemäß Figur 4 wird eine Kapillarwirkung durch einen Docht-Wendel-Verdampfer 20 erzeugt, d.h. mittels einer Heizwendel als Heizelement 22 und einem in der Heizwendel 22 angeordneten Docht 23.

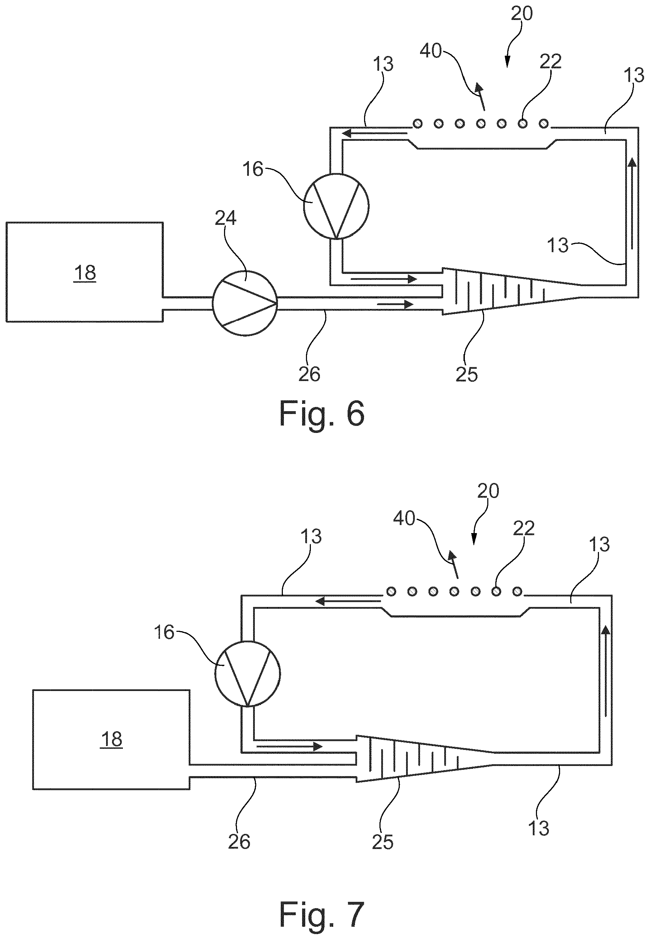

In der Ausführungsform gemäß Figuren 6 und 7 ist der Flüssigkeitsspeicher 18 nicht in die Kreislaufleitung 13 geschaltet, sondern separat von dieser angeordnet. Flüssigkeit wird aus dem Flüssigkeitsspeicher 18 über ein Verzweigungselement 25 in die Kreislaufleitung 13 nachgespeist. Das Verzweigungselement 25 hat drei Anschlüsse, nämlich zwei für Ein- und Ausgang der Ringleitung 13 und einen weiteren Eingang, der über eine separate Leitung 26 mit dem Flüssigkeitsspeicher 18 verbunden ist. Das Verzweigungselement 25 ist vorteilhaft als Mischer, insbesondere als statischer Mischer, beispielsweise als Labyrinthmischer ausgeführt.

In einer nicht gezeigten Ausführungsform können Mischer 25 und Verdampfer 20 bzw. Heizelement 22 in einem einheitlichen Mischer-Verdampfer-Element kombiniert werden.

Die Nachförderung von Flüssigkeit aus dem Flüssigkeitsspeicher 18 kann mittels einer weiteren Mikropumpe 24 erfolgen, siehe Figur 6. Das verdampfte Liquid wird durch die zweite Mikropumpe 24 aus dem Tank 18 nachgefüllt und in dem statischen Mischer 25 mit dem Umlaufmassenstrom in der Kreislaufleitung 13 vermischt, so dass eine möglichst homogene Flüssigkeitsmischung entsteht.

Die Nachförderpumpe 24 kann entfallen, siehe Figur 7, wenn eine selbsttätige Nachförderung beispielsweise durch Kapillarwirkung, Druckbeaufschlagung oder auf andere Weise erzielt wird.

Zusätzlich oder alternativ zu der Förderung per Kapillarwirkung oder Förderpumpe 24 können andere Fördermechanismen vorgesehen sein. Beispielsweise kann in den Ausführungsformen gemäß Figuren 3 und 7 eine Druckbeaufschlagung des Flüssigkeitsspeichers 18 vorgesehen sein. Erforderlich ist in jedem Fall eine Nachförderung der jeweils verdampften Flüssigkeitsmenge, ggf. unter Aufrechterhaltung der Zwangskonvektion in der Kreislaufleitung 13.

Die Verdampfereinheit 17 umfassend Verdampfer 20, Pumpe 16, ggf. Mischer und Verbindungsleitungen ist zur Vermeidung hoher thermischer Kapazitäten und damit verbundenem schlechtem Ansprechverhalten vorteilhaft möglichst klein ausgeführt. Der Verdampfer 20 und/oder die Verdampfereinheit sind daher vorzugsweise auf der Grundlage von MEMS-Technologie gefertigt und somit vorteilhaft ein Mikro-Elektro-Mechanisches System.

Die Verdampfungseinheit 29 ist so eingestellt, dass eine vorteilhafte Flüssigkeitsmenge im Bereich zwischen 1 µl und 20 µl, weiter vorzugsweise zwischen 2 µl und 10 µl, noch weiter vorzugsweise zwischen 3 µl und 5 µl, typischerweise 4 µl pro Zug des Konsumenten, zudosiert wird. Vorzugsweise kann die Flüssigkeitsmenge pro Zug einstellbar sein.

Die Dosierer/Verdampfer-Kombination kann vorteilhaft so eingestellt sein, dass überwiegend Flüssigkeitstropfen mit einem Durchmesser im Bereich zwischen 0,05 µm und 5 µm, bevorzugt zwischen 0,1 µm und 3 µm entstehen. Tröpfchengrößen im Bereich zwischen 0,05 und 5 MMAD (mass median aerodynamic diameter, massen-medianer aerodynamischer Durchmesser), vorzugsweise zwischen 0,1 und 3 MMAD, weiter vorzugsweise zwischen 0,5 und 2 MMAD, noch weiter vorzugsweise zwischen 0,7 und 1,5 MMAD, beispielsweise um ca. 1 MMAD können optimal sein. MMAD entspricht einer EU-Norm und wird in µm spezifiziert.

Die Steuerung der Umwälzeinrichtung 16 erfolgt durch die elektronische Steueranordnung 29. Um den Stromverbrauch durch die Umwälzeinrichtung 16 zu senken, kann es vorteilhaft sein, wenn die Umwälzeinrichtung 16 nicht permanent, sondern nur phasenweise läuft, da nach einer bestimmten Zeitdauer des Betriebes der Umwälzeinrichtung 16 eine im Wesentlichen vollständige Durchmischung der Flüssigkeit gegeben ist und weitere Umwälzung dann keinen signifikanten Vorteil mehr bringt. Beispielsweise kann die Umwälzeinrichtung 16 bei Feststellung eines Zuges durch den Konsumenten eingeschaltet werden. Die Umwälzeinrichtung 16 kann dann mit oder nach Beendigung des Zuges durch den Konsumenten ausgeschaltet werden. Dies kann entweder sofort oder mit einer vorbestimmten Zeitverzögerung geschehen. Beispielsweise kann die Umwälzeinrichtung 16 ausgeschaltet werden, wenn nach einer vorbestimmten Zeitdauer, die beispielsweise im Bereich zwischen 3 s und 60 s liegen kann, nicht ein erneuter Zug durch den Konsumenten festgestellt wird. Wenn der Inhalator 10 einen Ein-/Aus-Schalter aufweist, kann beim Einschalten des Inhalators 10 die Umwälzeinrichtung 16 mindestens für eine vorbestimmte Zeitdauer betrieben und dann ausgeschaltet werden, wenn nicht innerhalb der Zeitdauer ein erneuter Zug durch den Konsumenten festgestellt wird. Des Weiteren kann beim Ausschalten des Inhalators 10 die Umwälzeinrichtung 16 sofort oder mit einer vorbestimmten Verzögerung ausgeschaltet werden.

## Patentansprüche

1. Verdampfereinheit (17) für einen Inhalator, mit einem Verdampfer (20), der ein Heizelement (22) zum Verdampfen von durch den Verdampfer (20) geförderter Flüssigkeit aufweist, wobei die Verdampfereinheit (17) eine Kreislaufleitung (13) aufweist, in der der Verdampfer (20) angeordnet ist, wobei in der Kreislaufleitung (13) eine Umwälzeinrichtung (16) zum Umwälzen von Flüssigkeit durch die Kreislaufleitung (13) angeordnet ist, **dadurch gekennzeichnet, dass** zum Nachfördern von verdampfter Flüssigkeit ein Kapillarförderer, eine Förderpumpe (24) oder eine Druckbeaufschlagungseinrichtung vorgesehen ist.

2. Verdampfereinheit nach Anspruch 1, **dadurch gekennzeichnet, dass** die Umwälzeinrichtung (16) elektrisch angetrieben ist.

3. Verdampfereinheit nach Anspruch 1 oder 2, **dadurch gekennzeichnet, dass** die Umwälzeinrichtung (16) eine Pumpe, insbesondere eine Mikropumpe ist.

4. Verdampfereinheit nach einem der vorangehenden Ansprüche, **dadurch gekennzeichnet, dass** die Verdampfereinheit (17) einen Flüssigkeitsspeicher (18) aufweist.

5. Verdampfereinheit nach Anspruch 4, **dadurch gekennzeichnet, dass** der Flüssigkeitsspeicher (18) in der Kreislaufleitung (13) angeordnet ist.

6. Verdampfereinheit nach Anspruch 4, **dadurch gekennzeichnet, dass** die Verdampfereinheit ein Verzweigungselement (25) zum Zuführen von Flüssigkeit aus dem Flüssigkeitsspeicher (18) in die Kreislaufleitung (13) aufweist.

7. Verdampfereinheit nach Anspruch 6, **dadurch gekennzeichnet, dass** das Verzweigungselement (25) als Mischer, insbesondere als statischer Mischer, ausgebildet ist.

8. Verdampfereinheit nach einem der vorangehenden Ansprüche, **dadurch gekennzeichnet, dass** die Verdampfereinheit (17) ein einheitliches Mikro-Elektro-Mechanisches System ist.

9. Inhalator (10) mit einer Verdampfereinheit (17) nach einem der vorangehenden Ansprüche.

## Claims

1. Evaporator unit (17) for an inhaler with an evaporator (20) comprising a heating element (22) for vaporizing a liquid fed through said evaporator (20), wherein said evaporator unit (17) comprises a circulation line (13) in which said evaporator (20) is arranged, a circulation device (16) being arranged in said circulation line (13) for circulating the liquid through said circulation line (13), **characterized in that** a capillary feed, a feed pump (24) or a pressurizing device is provided for feeding vaporized liquid.

2. Evaporator unit according to claim 1, **characterized in that** said circulation device (16) is driven electrically.

3. Evaporator unit according to claim 1 or 2, **characterized in that** said circulation device (16) is a pump, in particular a micropump.

4. Evaporator unit according to one of the preceding claims, **characterized in that** said evaporator unit (17) comprises a liquid storage (18).

5. Evaporator unit according to claim 4, **characterized in that** said liquid storage (18) is arranged in said circulation line (13).

6. Evaporator unit according to claim 4, **characterized in that** said evaporator unit comprises a branching element (25) for feeding liquid from said liquid storage (18) into said circulation line (13).

7. Evaporator unit according to claim 6, **characterized in that** said branching element (25) is realized as mixer, in particular as static mixer.

8. Evaporator unit according to one of the preceding claims, **characterized in that** said evaporator unit (17) is a single microelectromechanical system.

9. Inhaler (10) having an evaporator unit (17) according to one of the preceding claims.

## Revendications

1. Unité d'évaporation (17) pour un inhalateur, comprenant un vaporisateur (20), qui comporte un élément chauffant (22) pour la vaporisation de liquide refoulé par le vaporisateur (20), l'unité d'évaporation (17) comportant une conduite de circulation (13), dans laquelle est disposé le vaporisateur (20), un moyen de circulation (16) étant disposé dans la conduite de circulation (13) pour faire circuler du liquide à travers la conduite de circulation (13), **caractérisée en ce qu'**un élément de refoulement capillaire, une pompe de refoulement (24) ou un moyen d'application de pression est prévu(e) pour refouler du liquide vaporisé.

2. Unité d'évaporation selon la revendication 1, **caractérisée en ce que** le moyen de circulation (16) est entraîné de manière électrique.

3. Unité d'évaporation selon la revendication 1 ou 2, **caractérisée en ce que** le moyen de circulation (16) est une pompe, en particulier une micropompe.

4. Unité d'évaporation selon l'une des revendications précédentes, **caractérisée en ce que** l'unité d'évaporation (17) comporte un réservoir à liquide (18).

5. Unité d'évaporation selon la revendication 4, **caractérisée en ce que** le réservoir à liquide (18) est disposé dans la conduite de circulation (13).

6. Unité d'évaporation selon la revendication 4, **caractérisée en ce que** l'unité d'évaporation comporte un élément de branchement (25) pour alimenter du liquide provenant du réservoir à liquide (18) dans la conduite de circulation (13).

7. Unité d'évaporation selon la revendication 6, **caractérisée en ce que** l'élément de branchement (25) est réalisé sous la forme d'un mélangeur, en particulier d'un mélangeur statique.

8. Unité d'évaporation selon l'une des revendications précédentes, **caractérisée en ce que** l'unité d'évaporation (17) est un microsystème électromécanique formant un tout.

9. Inhalateur (10) comprenant une unité d'évaporation (17) selon l'une des revendications précédentes.
